Europäisches Patentamt

European Patent Office

Office européen des brevets

(19)

(11) Veröffentlichungsnummer: **0 173 198**
**B1**

(12) **EUROPÄISCHE PATENTSCHRIFT**

(45) Veröffentlichungstag der Patentschrift:
**03.08.88**

(51) Int. Cl.⁴: **C 07 C 49/497,** C 07 C 49/463,
C 07 C 45/64, C 07 C 45/63

(21) Anmeldenummer: **85110340.8**

(22) Anmeldetag: **19.08.85**

(54) **Verfahren zur Herstellung von 2-Hydroxy-cyclohexanon-(1).**

(30) Priorität: **27.08.84  DE 3431415**

(43) Veröffentlichungstag der Anmeldung:
**05.03.86 Patentblatt 86/10**

(45) Bekanntmachung des Hinweises auf die Patenterteilung:
**03.08.88 Patentblatt 88/31**

(84) Benannte Vertragsstaaten:
**CH DE FR LI NL**

(56) Entgegenhaltungen:
**EP - A - 0 149 407**
**FR - A - 2 114 548**
**US - A - 3 303 224**

(73) Patentinhaber: **HOECHST AKTIENGESELLSCHAFT,
Postfach 80 03 20, D-6230 Frankfurt am Main 80 (DE)**

(72) Erfinder: **Tronich, Wolfgang, Dr., Adolf-Guckes-Weg 5,
D-6239 Eppstein/Taunus (DE)**
Erfinder: **Wykypiel, Werner, Dr., Egerstrasse 7,
D-6054 Rodgau (DE)**

## Beschreibung

Die vorliegende Erfindung liegt auf dem Gebiet der Herstellung von Zwischenprodukten.

2-Hydroxy-cyclohexanon-(1) ist technisch als cycloaliphatische Vorstufe zur Herstellung von Brenzkatechin von Interesse (s. bspw. Deutsche Offenlegungsschrift Nr. 2 155 561). Ferner dient es als Zwischenprodukt zur Herstellung von Tetrahydrocarbazol-Verbindungen, die wiederum als Vorstufen für substituierte Carbazole technisch interessant sind [s. bspw. Chem. Ber. 92, 2385 ff. (1959)].

Gemäss der Deutschen Offenlegungsschrift 2 155 561 kann die Synthese von 2-Hydroxy-cyclohexanon durch Hydrolyse von 2-Halogen-cyclohexanon erfolgen, wobei das 2-Chlor-cyclohexanon als eigentliche Ausgangsverbindung erwähnt und eingesetzt wird. Zwar ist auch die Verwendung von 2-Brom-cyclohexanon zur Herstellung von 2-Hydroxy-cyclohexanon beschrieben worden [s. Liebigs Ann. 448, 60 (1926)], jedoch hat die Verwendung des 2-Brom-Derivates in dieser Verfahrensweise gegenüber der 2-Chlor-Ausgangsverbindung keine offensichtlichen Vorteile.

Kennzeichnend für alle bisher bekannt gewordenen Herstellungsverfahren für 2-Hydroxy-cyclohexanon-(1) ist, dass das 2-Halogen-cyclohexanon-(1) nach seiner Herstellung in Substanz zwischenisoliert und in einem separaten Verfahrensschritt zum 2-Hydroxy-cyclohexanon hydrolysiert wird. Solch eine Verfahrensweise bringt jedoch mehrere Nachteile mit sich: So besitzt das 2-Chlor-cyclohexanon sehr unangenehme physiologische Eigenschaften [s. Helv. Chim. Acta 12, 10 (1929)], die umfangreiche und kostspielige Sicherheitsmassnahmen erforderlich machen und im Grunde sogar die Verwendung der isolierten Substanz in technischem Massstabe ausschliessen. Ebenso stellt der Umgang mit grösseren Mengen Halogen, wie gasförmigem Chlor und flüssigem Brom, hohe Anforderungen an die technische Ausstattung und an die Sicherheitsvorkehrungen des Produktionsbetriebes.

Es bestand somit die Aufgabe, ein Verfahren zur Herstellung von 2-Hydroxy-cyclohexanon aufzufinden, das die derzeit bestehenden Nachteile und Risiken beseitigt oder zumindestens auf ein technisch verantwortungsvolles Mass reduziert und das den heutigen Anforderungen an Sicherheit und Gewerbehygiene entspricht. Mit der vorliegenden Erfindung wurde solch ein Verfahren gefunden.

Die Erfindung betrifft ein Verfahren zur Herstellung von 2-Hydroxy-cyclohexanon durch Bromierung von Cyclohexanon in wässrig-organischem, bevorzugt in wässrigem Medium und anschliessende Hydrolyse des gebildeten 2-Brom-cyclohexanons mit einer alkalisch wirkenden Verbindung bei einem pH-Wert von oberhalb 7,5 und bei einer Temperatur bis zu 100 °C, das dadurch gekennzeichnet ist, dass die Bromierungsreaktion bei einer Temperatur bis zu 50 °C bei einem pH-Wert zwischen 0 und 4 durch Einwirkung eines Bromierungsagenz in mindestens äquivalenter Menge zum Cyclohexanon erfolgt, wobei das Bromierungsagenz ein stöchiometrisch äquivalentes Gemisch aus einem Halogenatsalz und Brom oder ein stöchiometrisch äquivalentes Gemisch aus einem Halogenatsalz und Bromwasserstoff in jeweils mindestens äquivalenten Mengen der Komponenten zum Cyclohexanon ist oder ein entsprechendes Gemisch dieser beiden Bromierungsagentien ist, und dass die anschliessende Hydrolyse des 2-Brom-cyclohexanons ohne dessen Zwischenisolierung durchgeführt wird.

Die Bromierungsreaktion erfordert pro Mol Cyclohexanon jeweils mindestens ⅙ Mol Halogenatsalz und ½ Mol Brom bzw. mindestens ⅓ Mol Halogenatsalz und 1 Mol Bromwasserstoffsäure. In der ersten Verfahrensvariante werden bei der Bromierungsreaktion in der Regel pro Mol Cyclohexanon 0,17 bis 0,2 Mol Halogenatsalz und 0,5 bis 0,6 Mol Brom, in der zweiten Verfahrensvariante 0,33 bis 0,4 Mol Halogenatsalz und 1,0 bis 1,2 Mol Bromwasserstoff verwendet.

Die Halogenatsalze sind insbesondere Alkalihalogenate, wie Alkalichlorate und Alkalibromate. Bevorzugt wird Natriumchlorat verwendet. Das Halogenatsalz dient als Oxidationsmittel zur Oxidation der Bromidionen bzw. der Bromwasserstoffsäure. In überraschender Weise erfolgt unter den gewählten Reaktionsbedingungen keine oxidative Zerstörung des Cyclohexanons bzw. des gebildeten 2-Brom-Derivates.

Die beiden Verfahrensvarianten können, wie aus der obigen Erfindungsdefinition hervorgeht, miteinander auch in kombinierter Form durchgeführt werden, wobei die beiden Bromierungsagenzien in summa in einer Menge vorliegen, die mindestens einem Bromierungsäquivalent, bezogen auf das Cyclohexanon, entspricht.

Die Bromierungsreaktion des Cyclohexanons zum 2-Brom-cyclohexanon verläuft exotherm; deren Reaktionstemperatur sollte 50 °C nicht überschreiten, weswegen sie vorteilhafterweise bei einer Temperatur zwischen 0 und 50 °C, bevorzugt zwischen 10 und 40 °C, insbesondere jedoch zwischen 15 und 25 °C durchgeführt wird. Der pH des wässrigen Reaktionsmediums sollte während der Bromierungsreaktion den Wert 4 nicht überschreiten; bevorzugt wird die Reaktion bei einem pH-Wert unterhalb 2, insbesondere bei einem pH-Wert zwischen 0 und 1 durchgeführt. Zur Einstellung des sauren pH-Bereiches bei der Bromierungsreaktion wird eine Mineralsäure verwendet, bevorzugt Schwefelsäure oder Salzsäure oder aber auch der als Ausgangsverbindung dienende Bromwasserstoff im Überschuss.

Die nachfolgende Hydrolyse des gebildeten 2-Brom-cyclohexanon erfolgt ohne vorherige Isolierung dieses Produktes im gleichen Reaktionsansatz nach Zugabe einer alkalisch wirkenden Verbindung, wie einem Alkalicarbonat und insbesondere einem Alkalihydroxid, bei einem pH-Wert von oberhalb 7,5, bevorzugt bei einem pH-Wert zwischen 8 und 12. Zur Hydrolysenreaktion wird die äquimolare Menge an der alkalisch wirkenden Verbindung benötigt; in der Regel wird sie in einem bis zu 10%igen Überschuss verwendet. Die Hydrolyse des 2-Brom-cyclohexanons zum 2-Hydroxy-cyclohexanon wird im allgemeinen bei einer Temperatur zwischen 10 und 100 °C durchgeführt; bevorzugt wird eine Hydrolysetemperatur zwischen 25 und 80 °C, insbesondere zwischen 45 und 55 °C gewählt.

Bei Durchführung des erfindungsgemässen Verfahrens in wässrig-organischem Medium dienen als organische Lösemittel solche, die mit Wasser mischbar sind und die gegenüber Brom und Halogenatsalzen unter den Reaktionsbedingungen des erfindungsgemässen Verfahrens inert sind. Solche organischen Lösungsmittel sind beispielsweise Alkanole von 1 bis 4 C-Atomen, bevorzugt hiervon Methanol.

Die vorliegende Erfindung wird somit bevorzugt gemäss der erstgenannten Verfahrensvariante in der Weise ausgeführt, dass man in wässrigem Medium, bevorzugt in rein wässrigem Medium, Cyclohexanon mit Natriumchlorat und Brom in einer Menge von mindestens ⅙ Mol Natriumchlorat und mindestens ½ Mol Brom pro Mol Cyclohexanon bei einem pH-Wert unterhalb von 2, vorzugsweise zwischen 0 und 1, und einer Temperatur zwischen 10 und 40 °C umsetzt, anschliessend im selben Reaktionsansatz ohne vorherige Isolierung des gebildeten 2-Brom-cyclohexanons eine alkalisch wirkende Verbindung, wie insbesondere Natriumhydroxid als wässrige Lösung, unter Einhaltung einer Temperatur von 25 bis 80 °C zugibt und einen pH-Wert zwischen 8 und 12 einstellt und innerhalb dieses pH-Bereiches die Hydrolyse bei einer Temperatur zwischen 25 und 80 °C zu Ende führt.

Bevorzugt wird die zweite Verfahrensvariante des erfindungsgemässen Verfahrens in der Weise ausgeführt, dass man in wässrigem Medium, bevorzugt in rein wässrigem Medium, Cyclohexanon bei einem pH-Wert unterhalb von 2, insbesondere zwischen 0 und 1, und einer Temperatur zwischen 10 und 40 °C mit mindestens ⅓ Mol Natriumchlorat und mindestens 1 Mol wässriger Bromwasserstoffsäure pro Mol Cyclohexanon umsetzt und das gebildete 2-Brom-cyclohexanon im selben Reaktionsansatz ohne vorherige Isolierung dieses Produktes nach Zugabe einer alkalisch wirkenden Verbindung, wie vorzugsweise Natriumhydroxid in Form einer wässrigen Lösung, bis zur Einstellung eines pH-Wertes zwischen 8 und 12 unter Einhaltung einer Temperatur zwischen 25 und 80 °C innerhalb dieses pH- und Temperaturbereiches hydrolysiert.

Beide Varianten des erfindungsgemässen Verfahrens haben den technischen Vorteil einer apparativ einfachen Ausführungsweise: Bei der erstgenannten Variante wird die effektiv gehandhabte Menge an flüssigem Brom auf die Hälfte derjenigen Menge üblicher Bromierungsverfahren reduziert. Apparativ und sicherheitstechnisch noch günstiger gestaltet sich jedoch die zweitgenannte Verfahrensvariante, bei der völlig auf die Handhabung von elementarem Brom verzichtet wird und bei der das zur Bromierungsreaktion erforderliche Brom erst durch Oxidation des Bromwasserstoffs durch das Halogenatsalz, wie Natriumchlorat, gebildet wird.

Das hierbei intermediär erzeugte Brom wird sofort durch die eigentliche Bromierungsreaktion verbraucht, so dass die stationäre Bromkonzentration in der Reaktionsmischung nur sehr gering ist. Man verfährt deshalb bei der zweiten Verfahrensvariante besonders vorteilhaft in der Weise, dass man entweder zu vorgelegtem Cyclohexanon, gegebenenfalls in einem mit Wasser mischbaren organischen Lösemittel gelöst, oder zu einem vorgelegten Zweiphasensystem Wasser/Cyclohexanon, dem ein mit Wasser mischbares organisches Lösemittel zugesetzt werden kann, eine wässrige Lösung des Halogenatsalzes und eine wässrige Lösung der Bromwasserstoffsäure, ggf. in deutlichem Überschuss zwecks Einstellung eines stark sauren pH-Wertes, in entsprechenden äquivalenten Anteilen zur Bildung des Broms zulaufen lässt oder dass man bevorzugt in ein vorgelegtes zweiphasiges Gemisch aus Cyclohexanon und einer wässrigen, ggf. stark sauren Lösung des Halogenatsalzes, ggf. unter Zusatz eines mit Wasser mischbaren organischen Lösemittels, die wässrige Lösung der Bromwasserstoffsäure, erforderlichenfalls im Überschuss zur Einstellung des stark sauren pH-Bereiches, zulaufen lässt.

Eine besonders vorteilhafte Ausführungsform der Verfahrensvarianten ist die, dass das als Agenz eingesetzte Alkalihalogenat, bevorzugt Natriumchlorat, in der erforderlichen Menge in wässriger Mineralsäure, bevorzugt Salzsäure oder Schwefelsäure, gelöst und mit der entsprechenden Menge an Cyclohexanon zusammengegeben wird und dass man in das so gebildete Zweiphasensystem unter Rühren die erforderliche Menge an Brom bzw. an Bromwasserstoff in wässriger Lösung zugibt.

Die nach der Hydrolyse des 2-Brom-cyclohexanons erhaltene wässrige Lösung des 2-Hydroxy-cyclohexanons kann zur Abtrennung und Isolierung des 2-Hydroxy-cyclohexanons in an und für sich bekannter Verfahrensweise aufgearbeitet werden. Bevorzugt wird jedoch das 2-Hydroxy-cyclohexanon direkt zur Weiterverarbeitung zu Folgeprodukten eingesetzt, so beispielsweise zur Herstellung von substituierten Tetrahydrocarbazolen.

Die nachfolgenden Beispiele dienen zur Erläuterung der Erfindung. Die Teile sind Gewichtsteile, und die Prozentangaben beziehen sich auf Gewichtsprozente, sofern nicht anders vermerkt.

Beispiel 1

23,4 Teile Natriumchlorat werden in 125 Teilen einer etwa 6%igen wässrigen Salzsäure gelöst, und 98 Teile Cyclohexanon werden zugesetzt. Unter Rühren gibt man innerhalb von etwa 60 min bei einer Temperatur von 20 bis 25 °C insgesamt 88 Teile Brom hinzu (die Reaktionstemperatur wird durch leichte Aussenkühlung gehalten). Man rührt noch kurze Zeit nach und lässt sodann langsam 146 Teile einer 33%igen wässrigen Natronlauge zulaufen, wobei man die Temperatur des Reaktionsgemisches auf bis zu 50 °C ansteigen lässt. Man rührt noch 2 h nach und stellt den Ansatz mit wenig verdünnter wässriger Salzsäure auf einen pH-Wert von 7 ein.

Es werden 482 Teile einer 21,5%igen wässrigen Lösung von 2-Hydroxy-cyclohexanon erhalten, was einer theoretischen Ausbeute von 90,3% an 2-Hydroxy-cyclohexanon entspricht.

Die wässrige Lösung enthält noch etwa 1,5% nicht umgesetztes Cyclohexanon (die Gehaltsbestimmung erfolgte durch gaschromatographische Analyse eines aliquoten Methylenchlorid-Extraktes der wässrigen Syntheselösung).

Beispiel 2

17,7 Teile Natriumchlorat werden in 125 Teilen einer etwa 6%igen wässrigen Salzsäure gelöst, und anschliessend werden 98 Teile Cyclohexanon zugesetzt. Unter Rühren gibt man innerhalb von 60 min unter Einhaltung einer Reaktionstemperatur von 20 bis 25 °C 80 Teile Brom hinzu. Man rührt noch kurze Zeit weiter und führt die Hydrolysenreaktion anschliessend gemäss der Verfahrensweise des Beispiels 1 durch.

Man erhält 468 Teile einer 20,5%igen wässrigen Lösung von 2-Hydroxy-cyclohexanon entsprechend einer theoretischen Ausbeute von 84% an 2-Hydroxy-cyclohexanon. Der Syntheseansatz enthält nach der Reaktion noch etwa 3% nicht umgesetztes Cyclohexanon.

Beispiel 3

Man löst 39 Teile Natriumchlorat in 75 Teilen Wasser und gibt anschliessend 98 Teile Cyclohexanon hinzu. Unter Rühren lässt man langsam unter Einhaltung einer Reaktionstemperatur zwischen 20 und 25 °C 141 Teile einer etwa 62%igen wässrigen Bromwasserstoffsäure zufliessen. Man rührt sodann noch eine kurze Zeit nach und versetzt den Ansatz anschliessend mit insgesamt 140 Teilen einer 33%igen wässrigen Natronlauge, wobei man die Innentemperatur auf etwa 50 °C ansteigen lässt. Es wird noch etwa zwei Stunden nachgerührt und der pH des Reaktionsansatzes anschliessend mit wenig verdünnter Salzsäure auf einen Wert von 7 gestellt. Es wird eine etwa 19%ige wässrige Lösung des 2-Hydroxy-cyclohexanons, entsprechend einer theoretischen Ausbeute von 82% an 2-Hydroxy-cyclohexanon, erhalten.

Der Reaktionsansatz enthält noch etwa 3% nicht umgesetztes Cyclohexanon.

Beispiel 4

Man verfährt gemäss den Angaben des Beispiels 1, gibt jedoch zuvor zur Lösung des Natriumchlorats in der wässrigen Salzsäure zusätzlich noch 30 Teile Methanol hinzu.

Man erhält eine wässrige Lösung von 2-Hydroxy-cyclohexanon in praktisch gleicher Ausbeute.

## Patentansprüche

1. Verfahren zur Herstellung von 2-Hydroxy-cyclohexanon durch Bromierung von Cyclohexanon in einem wässrigen oder wässrig-organischen Medium und anschliessende Hydrolyse des gebildeten 2-Brom-cyclohexanons mit einer alkalisch wirkenden Verbindung bei einem pH-Wert von oberhalb 7,5 und einer Temperatur von bis zu 100°C, dadurch gekennzeichnet, dass die Bromierungsreaktion bei einer Temperatur bis zu 50 °C und bei einem pH-Wert zwischen 0 und 4 durch Einwirkung eines Bromierungsagenz in mindestens äquivalenter Menge zum Cyclohexanon erfolgt, wobei das Bromierungsagenz ein stöchiometrisch äquivalentes Gemisch aus einem Halogenatsalz und Brom oder ein stöchiometrisch äquivalentes Gemisch aus einem Halogenatsalz und Bromwasserstoff in jeweils mindestens äquivalenten Mengen der Komponenten ist oder ein entsprechendes Gemisch dieser beiden Bromierungsagentien ist, und dass die anschliessende Hydrolyse des 2-Brom-Cyclohexanons ohne dessen Zwischenisolierung durchgeführt wird.

2. Verfahren nach Anspruch 1, dadurch gekennzeichnet, dass die Bromierungsreaktion unter Einwirkung von jeweils mindestens ⅙ Mol Halogenatsalz und ½ Mol Brom pro Mol Cyclohexanon bzw. mindestens ⅓ Mol Halogenatsalz und 1 Mol Bromwasserstoff pro Mol Cyclohexanon erfolgt.

3. Verfahren nach Anspruch 1 oder 2, dadurch gekennzeichnet, dass die Bromierung bei einem pH-Wert unterhalb 2 durchgeführt wird.

4. Verfahren nach Anspruch 1, 2 oder 3, dadurch gekennzeichnet, dass als Halogenatsalz ein Alkalihalogenat, vorzugsweise Natriumchlorat, eingesetzt wird.

5. Verfahren nach einem der Ansprüche 1 bis 4, dadurch gekennzeichnet, dass man bei der Bromierungsreaktion die Bromwasserstoffsäure in Form einer wässrigen Lösung zu einer vorgelegten Mischung aus Cyclohexanon und einer wässrigen, gegebenenfalls sauren Natriumchloratlösung zugibt.

6. Verfahren nach einem der Ansprüche 1 bis 5, dadurch gekennzeichnet, dass die Bromierung bei einer Reaktionstemperatur von 15 bis 25 °C durchgeführt wird.

7. Verfahren nach einem der Ansprüche 1 bis 6, dadurch gekennzeichnet, dass nach der Bromierungsreaktion die Hydrolyse zum 2-Hydroxy-cyclohexanon bei einer Temperatur zwischen 25 und 80 °C und einem pH-Wert zwischen 8 und 10 erfolgt.

8. Verfahren nach einem der Ansprüche 1 bis 7, dadurch gekennzeichnet, dass die Bromierung in einem wässrigen Medium durchgeführt wird.

9. Verfahren nach einem der Ansprüche 1 bis 7, dadurch gekennzeichnet, dass bei Durchführung der Bromierungsreaktion in einem wässrig-organischen Reaktionsmedium dieses ein wässrig-alkanolisches Medium ist.

## Claims

1. A process for the preparation of 2-hydroxy-cyclohexanone by brominating cyclohexanone in an aqueous or aqueous-organic medium and subsequently hydrolysing the resulting 2-bromo-cyclohexanone with an alkaline compound at a pH above 7.5 and at a temperature of up to 100 °C, wherein the bromination reaction is carried out at a temperature of up to 50 °C and at a pH of between 0 and 4 by treatment with a brominating agent in an amount at least equivalent to the cyclohexanone, the brominating agent being a stoichiometrically equivalent mixture of a halogenate salt and bromine or a stoichiometrically equivalent mixture of a halogenate salt and hydrogen bromide, in each case in at least equivalent amounts of the components, or a corresponding mixture of both these brominating agents, and wherein the subsequent hydrolysis of the 2-bromo-cyclohexanone is carried out without intermediate isolation thereof.

2. The process as claimed in claim 1, wherein the bromination reaction is carried out with at least ⅙ mole of halogenate salt and ½ mole of bromine per mole of cyclohexanone or at least ⅓ mole of halogenate salt and 1 mole of hydrogen bromide per mole of cyclohexanone.

3. The process as claimed in claim 1 or 2, wherein the bromination is carried out at a pH below 2.

4. The process as claimed in claim 1, 2 or 3, wherein the halogenate salt employed is an alkali metal halogenate, preferably sodium chlorate.

5. The process as claimed in any of claims 1 to 4, wherein, in carrying out the bromination reaction, the hydrobromic acid is added in the form of an aqueous solution to a mixture of cyclohexanone and an aqueous, optionally acidic, sodium chlorate solution.

6. The process as claimed in any of claims 1 to 5, wherein the bromination is carried out at a reaction temperature of from 15 to 25 °C.

7. The process as claimed in any of claims 1 to 6, wherein, following the bromination reaction, the hydrolysis to give 2-hydroxy-cyclohexanone is carried out at a temperature of between 25 and 80 °C and a pH of between 8 and 10.

8. The process as claimed in any of claims 1 to 7, wherein the bromination is carried out in an aqueous medium.

9. The process as claimed in any of claims 1 to 7, wherein the aqueous-organic reaction medium in which the bromination reaction is carried out is an aqueous-alkanolic medium.

## Revendications

1. Procédé de préparation de la 2-hydroxy-cyclohexanone par bromation de la cyclohexanone dans un milieu aqueux ou hydro-organique, suivie de l'hydrolyse de la 2-bromo-cyclohexanone formée avec un composé à action alcaline, à un pH supérieur à 7,5 et à une température pouvant s'élever jusqu'à 100 °C, procédé caractérisé en ce qu'on effectue la réaction de bromation à une température pouvant s'élever jusqu'à 50 °C, à un pH compris entre 0 et 4, avec un agent de bromation en quantité au moins équivalente par rapport à la cyclohexanone, l'agent de bromation étant un mélange en équivalents stoechiométriques d'un halogénate minéral et de brome ou un mélange d'équivalents stoechiométriques d'un tel halogénate et de bromure d'hydrogène, dans les deux cas en quantités au moins équivalentes des deux composants du mélange par rapport à la cyclohexanone, ou bien un mélange de ces deux agents de bromation, et on effectue ensuite l'hydrolyse de la 2-bromo-cyclohexanone sans l'avoir isolée.

2. Procédé selon la revendication 1, caractérisé en ce que l'on effectue la réaction de bromation avec au moins ⅙ de mole de l'halogénate et au moins ½ mole de brome par mole de cyclohexanone, ou avec au moins ⅓ de mole de l'halogénate et 1 mole au moins de bromure d'hydrogène par mole de cyclohexanone.

3. Procédé selon la revendication 1 ou 2, caractérisé en ce que l'on effectue la bromation à un pH inférieur à 2.

4. Procédé selon la revendication 1, 2 ou 3, caractérisé en ce que l'on utilise un halogénate de métal alcalin, de préférence le chlorate de sodium.

5. Procédé selon l'une quelconque des revendications 1 à 4, caractérisé en ce que l'on effectue la réaction de bromation en ajoutant une solution aqueuse de l'acide bromhydrique à un mélange de la cyclohexanone et d'une solution aqueuse éventuellement acide de chlorate de sodium.

6. Procédé selon l'une quelconque des revendications 1 à 5, caractérisé en ce que l'on effectue la bromation à une température comprise entre 15 et 25 °C.

7. Procédé selon l'une quelconque des revendications 1 à 6, caractérisé en ce que, après la réaction de bromation, on effectue l'hydrolyse en 2-hydroxy-cyclohexanone à une température de 25 à 80 °C et à un pH compris entre 8 et 10.

8. Procédé selon l'une quelconque des revendications 1 à 7, caractérisé en ce que l'on effectue la bromation dans un milieu aqueux.

9. Procédé selon l'une quelconque des revendications 1 à 7, caractérisé en ce que, quand on effectue la réaction de bromation dans un milieu hydro-organique, ce milieu est un milieu hydro-alcanolique.